# EUROPÄISCHE PATENTANMELDUNG

(11) **EP 2 893 980 A1**
(43) Veröffentlichungstag der Anmeldung: **15.07.2015**
(21) Anmeldenummer: 14199576.1
(22) Anmeldetag: 22.12.2014
(51) Int. Cl.: B01L 3/00, B01L 7/00, C12Q 1/68

(54) **Mikrofluidisches System sowie Verfahren zum Analysieren einer Probe biologischen Materials**

(30) Priorität: 14.01.2014 DE 102014200467
(71) Anmelder: ROBERT BOSCH GMBH, 70442 Stuttgart (DE)
(72) Erfinder: Hoffmann, Jochen, 71229 Leonberg (DE)

(57) **Zusammenfassung**

Die Erfindung betrifft ein mikrofluidisches System zum Analysieren einer Probe biologischen Materials. Das mikrofluidische System weist eine Probenbearbeitungsvorrichtung (120) aus einem Polymermaterial auf. Hierbei weist die Probenbearbeitungsvorrichtung (120) eine Amplifikationskammer (210) für Amplifikationsreaktionen an Zielmolekülen der Probe und eine fluidisch mit der Amplifikationskammer (210) verbundene Nachweiskammer (260) mit einer Mehrzahl von Kavitäten (265) zum Ausführen von Nachweisreaktionen an den amplifizierten Zielmolekülen auf. Dabei sind die Nachweiskammer (260) und die Kavitäten (265) für eine Verteilung der Zielmoleküle der Probe auf die Kavitäten (265) ausgeformt. Auch weist das mikrofluidische System eine mit der Probenbearbeitungsvorrichtung (120) koppelbare mikrofluidische Kartusche zum Vorbereiten der Probe und Zuführen der Zielmoleküle und von Stoffen für das Analysieren der Probe zu der Probenbearbeitungsvorrichtung (120) auf.

## Beschreibung

### Stand der Technik

Die vorliegende Erfindung bezieht sich auf ein mikrofluidisches System zum Analysieren einer Probe biologischen Materials und auf ein Verfahren zum Analysieren einer Probe biologischen Materials.

Mikrofluidische Diagnosesysteme wie Chiplabore bzw. Labs-on-Chip (LoC) erlauben eine miniaturisierte und integrierte Durchführung komplexer fluidischer Arbeitsabläufe insbesondere für den spezifischen Nachweis verschiedenster Moleküle. Die DE 10 2009 035 270 A1 beschreibt einen Einweg-Multiplex-Polymerase-Kettenreaktions-Chip und ein Polymerase-Kettenreaktionsgerät.

### Offenbarung der Erfindung

Vor diesem Hintergrund werden mit dem hier vorgestellten Ansatz ein mikrofluidisches System zum Analysieren einer Probe biologischen Materials und ein Verfahren zum Analysieren einer Probe biologischen Materials gemäß den Hauptansprüchen vorgestellt. Vorteilhafte Ausgestaltungen ergeben sich aus den jeweiligen Unteransprüchen und der nachfolgenden Beschreibung.

Gemäß Ausführungsformen der Erfindung kann insbesondere ein mikrofluidischer Chip für eine Kombination von Amplifikations- und Nachweisreaktionen bereitgestellt werden. Insbesondere können gemäß Ausführungsformen der vorliegenden Erfindung geeignete Strukturen und Prozessabläufe für eine Kombination von Amplifikations- und Nachweisreaktionen auf einem polymeren Mehrschichtsystem bereitgestellt werden. Im Speziellen kann beispielsweise auf einem einzigen Chip ermöglicht werden, Zielmoleküle einer Probe zu amplifizieren und die amplifizierten Zielmoleküle, beispielsweise voramplifizierte Nukleinsäuren, auf viele separate Kavitäten bzw. Vertiefungen bzw. Reaktionskammern zu verteilen. In diesen Vertiefungen können dann jeweils eigenständige, parallele Nachweisreaktionen durchgeführt werden.

Vorteilhafterweise kann eine amplifizierte Probe bzw. ein Voramplifikat durch einfaches Überführen in eine Kammer, die eine Mehrzahl von Kavitäten enthält, insbesondere spitzwinklige Vertiefungen, auf viele weitere Reaktionskammern verteilt werden. Eine solche Aliquotierung oder Molekülverteilung kann eine Verbesserung der Nachweisgrenze ermöglichen. Für diese Aliquotierung oder Molekülverteilung werden zudem keine Ventile benötigt. Daher können eine Fehleranfälligkeit und ein Platzbedarf des mikrofluidischen Systems verringert werden. Insbesondere kann ein Aliquot oder eine Teilmenge an Molekülen aufgrund der Voramplifikation mehrere DNA-Abschnitte aufweisen. Gemäß Ausführungsformen der vorliegenden Erfindung können somit Reaktionen verschachtelt ausgeführt werden, wobei durch Voramplifikation der Zielmoleküle genügend Material für Einzelnachweisreaktionen erzeugt werden kann. Würde beispielsweise ein zu untersuchendes Probenmaterial, das sehr wenige Zielmoleküle enthält, direkt auf mehrere Reaktionskammern verteilt, bestünde die Gefahr, dass die einzelnen Kammern zu wenig Ausgangsmaterial für Nachweisreaktionen enthalten.

Zudem können eine Integration und Realisierung vieler Assays/Biocontents erreicht werden. So gibt es beispielsweise mehr Assays, bei denen beispielsweise DNA-Moleküle direkt über eine single-plex bis quad-plex Polymerase-Kettenreaktion (PCR, Polymerase Chain Reaction) nachgewiesen werden, als in einer Kombination aus multiplex-PCR plus anschließender Mikroarrayanalyse. Es kann gemäß Ausführungsformen der vorliegenden Erfindung insbesondere auf ein DNA-Mikroarray für einen Nachweis unterschiedlicher DNA-Moleküle verzichtet werden. Dadurch können auch Anforderungen an eine optische Detektionseinheit in punkto Auflösung gesenkt werden und kann sich eine Prozesszeit verkürzen. So kann insbesondere eine Verknüpfung aus einer (Vor-) Amplifikationsreaktion mit einer digitalen PCR erreicht werden. Eine digitale PCR hat eine Einzelmolekülauflösung und ermöglicht damit besondere Anwendungen beispielsweise aus dem Bereich der nichtinvasiven, pränatalen Diagnostik und der Analyse von zirkulierenden Tumorzellen.

Durch Kombination von biochemischen Reaktionen können beispielsweise viele verschiedene genetische Motive aus einer kleinen Menge an Nukleinsäuren in einer Probe bestimmt werden. Zunächst kann die Menge an Nukleinsäuren im Rahmen einer Amplifikationsreaktion erhöht werden. Dabei kann genügend Ausgangmaterial für eine Identifikation von verschiedenen Nukleinsäuren mittels einer anschließenden Nachweisreaktion erzeugt werden. Hierfür kann das Amplifikat auf mehrere separate Reaktionskammern bzw. Kavitäten verteilt werden. In jeder einzelnen Kavität kann dann beispielsweise ein genetisches Motiv mit einer geeigneten Nachweisreaktion identifiziert werden. Durch starke Miniaturisierung der Kavitäten kann das Voramplifikat in eine große Zahl von Vertiefungen eingefüllt werden. Dabei können sich die voramplifizierten Nukleinsäuren statistisch verteilen, sodass in einer Vertiefung entweder keine oder eine Nukleinsäure zu liegen kommt. Eine Nachweisreaktion kann dann für einzelne genetische Motive diskrete, digitale Signale beispielsweise im Rahmen einer digitalen PCR liefern. Von einer Anzahl an Signalen kann auf eine absolute Menge an genetischen Motiven geschlossen werden. Je mehr Zyklen eine Amplifikationsreaktion umfasst, desto mehr Zielmoleküle können gebildet werden und desto mehr Moleküle können in die Kavitäten gelangen. Es können einer Kavität so viele Moleküle zugeführt werden, dass in jeder Kavität jeder DNA-Abschnitt der Probe nachgewiesen werden kann, also insbesondere mindestens ein Molekül jeder in den Kavitäten nachweisbaren Sorte.

Ein mikrofluidisches System zum Analysieren einer Probe biologischen Materials weist folgende Merkmale auf:
eine Probenbearbeitungsvorrichtung aus einem Polymermaterial, wobei die Probenbearbeitungsvorrichtung eine Amplifikationskammer für Amplifikationsreaktionen an Zielmolekülen der Probe und eine fluidisch mit der Amplifikationskammer verbundene Nachweiskammer mit einer Mehrzahl von Kavitäten zum Ausführen von Nachweisreaktionen an den amplifizierten Zielmolekülen aufweist, wobei die Nachweiskammer und die Kavitäten für eine Verteilung der Zielmoleküle der Probe auf die Kavitäten ausgeformt sind; Für den Fall, dass die Amplifikationsreaktion eine PCR ist, gilt für die Menge an generierten Zielmolekülen *N* in Abhängigkeit der PCR-Zyklen *n* und der Amplifikationseffizienz *E: N = N₀*(1+E)ⁿ,* wobei *n* = 1 - 20 und *E* = 0,5 - 1 und *N₀* die Anfangskonzentration an Zielmolekülen ist. Für die Verteilung der Zielmoleküle entscheidet die Zyklenzahl *n,* ob *n* < *x* oder n > x (*x* = Anzahl an Kavitäten in der Nachweiskammer) ist. Für die hier beschriebene Erfindung ist es wichtig, dass n > x ist, und x soll größer gleich der Anzahl der in den Kavitäten nachweisbaren Molekülen sein, und
eine mit der Probenbearbeitungsvorrichtung koppelbare mikrofluidische Kartusche zum Vorbereiten der Probe und Zuführen der Zielmoleküle und von Stoffen für das Analysieren der Probe zu der Probenbearbeitungsvorrichtung.

Bei dem mikrofluidischen System kann es sich um ein analytisches System handeln, insbesondere ein mikrofluidisches Lab-on-Chip-System bzw. Chiplabor-System zur medizinischen Diagnostik, mikrobiologischen Diagnostik oder Umweltanalytik. Als Probe kann eine zu analysierende Flüssigkeit, typischerweise eine flüssige oder verflüssigte Patientenprobe, z. B. Blut, Urin, Stuhl, Sputum, Liquor, Lavage, ein ausgespülter Abstrich oder eine verflüssigte Gewebeprobe, oder eine Probe eines nichtmenschlichen Materials bezeichnet werden. In der Probe enthaltene Zellen können beispielsweise menschliche Zellen, z. B. Blutzellen oder dergleichen, tierische Zellen oder pathogene Zellen bzw. Pathogene, z. B. Viren oder Mikroorganismen, wie z. B. Bakterien oder Pilze, umfassen, die DNA und/oder RNA, d. h. Nukleinsäuremoleküle, als Zielmoleküle aufweisen. Bei der Probenbearbeitungsvorrichtung kann es sich um einen Chip, insbesondere mit mehreren Schichten, aus einem Polymermaterial handeln. Die Mehrzahl von Kavitäten können in der Nachweiskammer angeordnet sein. Die Mehrzahl von Kavitäten können in einem Array angeordnet sein. Die Kavitäten können Abmessungen sowie eine Form aufweisen, die geeignet sind, um die Verteilung der Zielmoleküle auf die Kavitäten zu ermöglichen. Somit können Dimension einer Kavität größer als Dimensionen eines Moleküls und sein. Die mikrofluidische Kartusche kann einen Abschnitt zum Einbringen der Probe in das mikrofluidische System, vorgelagerte Reagenzien und eine Aktuierungseinheit aufweisen, um der Amplifikationskammer und der Nachweiskammer mit den Kavitäten die Zielmoleküle der Probe und Stoffe für das Analysieren der Probe zuzuführen. Ein Aufschluss von Zellen der Probe und ein Aufreinigen der Zielmoleküle der Probe kann mittels geeigneter Techniken auf der mikrofluidischen Kartusche realisiert werden. Die Probenbearbeitungsvorrichtung kann ausgebildet sein, um aufgereinigte Zielmoleküle aus der Probe zu analysieren.

Gemäß einer Ausführungsform können die Kavitäten ausgebildet sein, um ein kapillares Befüllen derselben mit den Zielmolekülen der Probe zu bewirken. Dabei kann die Probenbearbeitungsvorrichtung innerhalb der Kavitäten eine hydrophile Oberfläche und außerhalb der Kavitäten eine hydrophobe Oberfläche aufweisen. Eine solche Ausführungsform bietet den Vorteil, dass eine wässrige Zielmoleküllösung in den Kavitäten ein Energieminimum einnimmt und somit bevorzugt die Kavitäten benetzt bzw. einfacher in die Kavitäten eingebracht werden kann.

Dabei können die Kavitäten spitzwinklig zulaufend ausgeformt sein. Insbesondere können die Kavitäten einen Öffnungswinkel zwischen 5 Grad und 90 Grad aufweisen. Die Kavitäten können somit eine Geometrie aufweisen, die eine kapillare Befüllung mit den Zielmolekülen der Probe begünstigt.

Auch kann die Probenbearbeitungsvorrichtung eine Zufuhröffnung, ein Einlassventil, ein Verbindungsventil, ein Auslassventil und eine Abfuhröffnung aufweisen. Hierbei kann die Amplifikationskammer über das Einlassventil mit der Zufuhröffnung verbunden sein. Dabei kann die Amplifikationskammer über das Verbindungsventil mit der Nachweiskammer verbunden sein. Hierbei kann die Nachweiskammer über das Auslassventil mit der Abfuhröffnung verbunden sein. Auch kann die Probenbearbeitungsvorrichtung eine erste Ausgangsöffnung und ein erstes Ausgangsventil aufweisen, wobei die Amplifikationskammer über das erste Ausgangsventil mit der ersten Ausgangsöffnung verbunden sein kann. Die erste Ausgangsöffnung und das erste Ausgangsventil können ausgebildet sein, um für die Amplifikationskammer ein Entlüften, Befüllen und zusätzlich oder alternativ Ablassen von Resten und/oder Überschüssen zu ermöglichen. Die Zufuhröffnung, die Abfuhröffnung und/oder die erste Ausgangsöffnung können jeweils an einen Kanal angrenzen oder als ein Kanal ausgeformt sein. Das Einlassventil, das Verbindungsventil, das Auslassventil und/oder das erste Ausgangsventil können jeweils in einem Kanal bzw. einem Bereich eines Kanals angeordnet sein. Eine solche Ausführungsform bietet den Vorteil, dass in dem mikrofluidischen System ein Ablauf von Vorbereitungsreaktionen und Analysereaktionen sowie ein Fluidtransport einfach, definiert und effizient gestaltet werden können.

Ferner kann die Probenbearbeitungsvorrichtung eine fluidisch mit der Nachweiskammer verbundene Speicherkammer zum Speichern eines Spülmediums für die Nachweiskammer aufweisen. Zudem kann die Probenbearbeitungsvorrichtung eine Spülmedienzufuhröffnung, ein Spülmedieneinlassventil und ein weiteres Verbindungsventil aufweisen. Hierbei kann die Speicherkammer über das Spülmedieneinlassventil mit der Spülmedienzufuhröffnung verbunden sein. Ferner kann die Speicherkammer über das weitere Verbindungsventil mit der Nachweiskammer verbunden sein. Auch kann die Probenbearbeitungsvorrichtung eine zweite Ausgangsöffnung und ein zweites Ausgangsventil aufweisen, wobei die Speicherkammer über das zweite Ausgangsventil mit der zweiten Ausgangsöffnung verbunden sein kann. Die zweite Ausgangsöffnung und das zweite Ausgangsventil können ausgebildet sein, um für die Speicherkammer ein Entlüften, Befüllen und zusätzlich oder alternativ Ablassen von Resten und/oder Überschüssen zu ermöglichen. Die Spülmedienzufuhröffnung und/oder die zweite Ausgangsöffnung können jeweils an einen Kanal angrenzen oder als ein Kanal ausgeformt sein. Das Spülmedieneinlassventil, das weitere Verbindungsventil und/oder das zweite Ausgangsventil können jeweils in einem Kanal bzw. einem Bereich eines Kanals angeordnet sein. Eine solche Ausführungsform führt ebenfalls zu einer einfachen, definierten und effizienten Vorbereitung und Analyse mittels des mikrofluidischen Systems.

Ein Verfahren zum Analysieren einer Probe biologischen Materials weist folgende Schritte auf:
Bereitstellen einer Probenbearbeitungsvorrichtung aus einem Polymermaterial, wobei die Probenbearbeitungsvorrichtung eine Amplifikationskammer und eine fluidisch mit der Amplifikationskammer verbundene Nachweiskammer mit einer Mehrzahl von Kavitäten aufweist, wobei die Nachweiskammer und die Kavitäten für eine Verteilung der Zielmoleküle der Probe auf die Kavitäten ausgeformt sind;
Zuführen der Zielmoleküle in die Amplifikationskammer;
Durchführen von Amplifikationsreaktionen an den in die Amplifikationskammer zugeführten Zielmolekülen;
Überführen der amplifizierten Zielmoleküle von der Amplifikationskammer in die Nachweiskammer, wobei die Zielmoleküle der Probe auf die Kavitäten verteilt werden; und

Ausführen von parallelen Nachweisreaktionen an den amplifizierten, in die Kavitäten der Nachweiskammer überführten Zielmolekülen .

Das Verfahren kann in Verbindung mit einer Ausführungsform des vorstehend genannten mikrofluidischen Systems vorteilhaft ausgeführt werden, um eine Probe biologischen Materials zu analysieren. Der Schritt des Zuführens und zusätzlich oder alternativ der Schritt des Überführens können durch ein Steuern von Ventilen der Probenbearbeitungsvorrichtung ausgeführt werden.

Gemäß einer Ausführungsform kann im Schritt des Durchführens eine Amplifikationsreaktion unter Verwendung von Wärme ausgeführt werden. Auch können im Schritt des Ausführens eine Mehrzahl von Nachweisreaktionen unter Verwendung von Wärme ausgeführt werden. Hierbei kann im Schritt des Durchführens eine Amplifikationsreaktion mittels zumindest einer Temperiereinrichtung ausgeführt werden. Dabei können im Schritt des Ausführens die Mehrzahl von Nachweisreaktionen mittels zumindest einer Temperiereinrichtung ausgeführt werden. Hierbei kann zumindest eine Temperiereinrichtung mit der Amplifikationskammer und/oder mit der Nachweiskammer bzw. den Kavitäten thermisch gekoppelt sein. Insbesondere können im Schritt des Ausführens die Mehrzahl von Nachweisreaktionen durch ein Durchlaufen von Temperaturzyklen ausgeführt werden. Eine solche Ausführungsform bietet den Vorteil, dass auch eine große Anzahl von parallelen Nachweisreaktionen beispielsweise mittels lediglich einer Temperiereinrichtung ausgeführt werden können. Das thermische Verfahrensprinzip ermöglicht eine möglichst gleichmäßige und parallele Einwirkung auf die Mehrzahl von Kavitäten bei einfacher und günstiger Realisierbarkeit.

Auch kann im Schritt des Zuführens zumindest ein Amplifikationshilfsstoff mit den Zielmolekülen in die Amplifikationskammer zugeführt werden. Eine solche Ausführungsform bietet den Vorteil, dass die Amplifikation durch einen solchen Hilfsstoff vereinfacht und gegebenenfalls beschleunigt werden kann.

Ferner kann das Verfahren zwischen dem Schritt des Überführens und dem Schritt des Ausführens von Nachweisreaktionen einen Schritt des Einleitens eines Spülmediums in die Nachweiskammer aufweisen, um die mit den Zielmolekülen befüllten Kavitäten für die Nachweisreaktionen abzudecken. Im Schritt des Einleitens kann das Spülmedium aus einer fluidisch mit der Nachweiskammer verbundenen Speicherkammer in die Nachweiskammer eingeleitet werden. Beispielsweise kann es sich bei dem Spülmedium um ein Öl oder dergleichen handeln. Eine solche Ausführungsform bietet den Vorteil, dass die Nachweisreaktionen genauere und sicherere Ergebnisse liefern können, wenn die Kavitäten mit den darin aufgenommenen Zielmolekülen durch das Spülmedium gedeckelt sind und somit die Moleküle während der Nachweisreaktionen zuverlässig vereinzelt bleiben können.

Zudem kann das Verfahren vor dem Schritt des Überführens einen Schritt des Anordnens zumindest eines Nachweishilfsstoffs in den Kavitäten aufweisen. Insbesondere können im Schritt des Anordnens Hilfsstoffe für die Nachweisreaktionen an den Zielmolekülen der Probe in den Kavitäten vorgelagert bzw. vorgelegt werden. Beispielsweise kann es sich bei einem Nachweishilfsstoff um einen Primer oder dergleichen handeln. Eine solche Ausführungsform bietet den Vorteil, dass die Nachweisreaktionen durch eine solche Vorbereitung der Kavitäten unterstützt und erleichtert werden können und die Analyse beschleunigt werden kann.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form einer Vorrichtung kann die der Erfindung zugrunde liegende Aufgabe schnell und effizient gelöst werden.

Unter einer Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Von Vorteil ist auch ein Computerprogrammprodukt mit Programmcode, der auf einem maschinenlesbaren Träger wie einem Halbleiterspeicher, einem Festplattenspeicher oder einem optischen Speicher gespeichert sein kann und zur Durchführung und/oder Ansteuerung der Schritte des Verfahrens nach einer der vorstehend beschriebenen Ausführungsformen verwendet wird, insbesondere wenn das Programmprodukt auf einem Computer oder einer Vorrichtung ausgeführt wird.

Der hier vorgestellte Ansatz wird nachstehend anhand der beigefügten Zeichnungen beispielhaft näher erläutert. Es zeigen:
Fig. 1 ein schematisches Blockdiagramm eines mikrofluidischen Systems gemäß einem Ausführungsbeispiel der vorliegenden Erfindung;
Fig. 2 eine schematische Darstellung einer Probenbearbeitungsvorrichtung gemäß einem Ausführungsbeispiel der vorliegenden Erfindung; und
Fig. 3 ein Ablaufdiagramm eines Verfahrens gemäß einem Ausführungsbeispiel der vorliegenden Erfindung.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

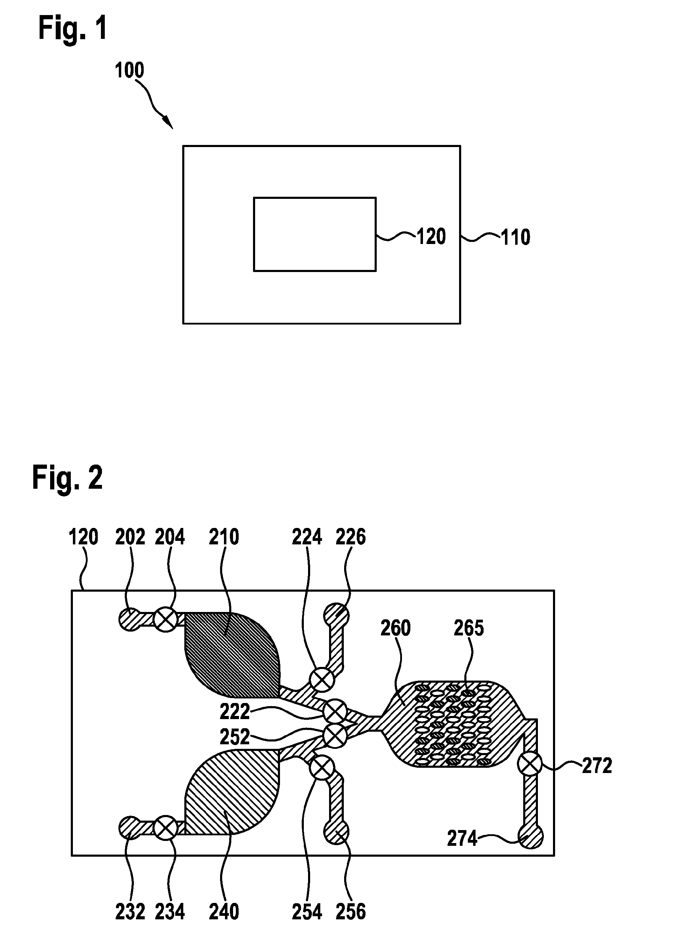

Fig. 1 zeigt ein schematisches Blockdiagramm eines mikrofluidischen Systems 100 zum Analysieren einer Probe biologischen Materials gemäß einem Ausführungsbeispiel der vorliegenden Erfindung. Von dem mikrofluidischen System 100 sind in Fig. 1 eine mikrofluidische Kartusche 110 und eine Probenbearbeitungsvorrichtung 120 gezeigt. Die Probenbearbeitungsvorrichtung 120 ist in der Darstellung von Fig. 1 mit der mikrofluidischen Kartusche 110 gekoppelt.

Bei dem mikrofluidischen System 100 handelt es sich um ein analytisches System, insbesondere ein mikrofluidisches Lab-on-Chip-System bzw. Chiplabor-System zur medizinischen Diagnostik, mikrobiologischen Diagnostik oder Umweltanalytik. Die Probe weist hierbei eine zu analysierende Flüssigkeit auf, insbesondere typischerweise eine flüssige oder verflüssigte Patientenprobe, z. B. Blut, Urin, Stuhl, Sputum, Liquor, Lavage, ein ausgespülter Abstrich oder eine verflüssigte Gewebeprobe, oder eine Probe eines nichtmenschlichen Materials. In der Probe enthaltene Zellen umfassen menschliche Zellen, beispielsweise Blutzellen oder dergleichen, tierische Zellen oder pathogene Zellen bzw. Pathogene, wie Viren oder Mikroorganismen, beispielsweise Bakterien oder Pilze, die DNA und zusätzlich oder alternativ RNA, also Nukleinsäuremoleküle, als Zielmoleküle aufweisen.

Die mikrofluidische Kartusche 110 und die Probenbearbeitungsvorrichtung 120 sind miteinander koppelbar ausgeformt. Auch wenn es in Fig. 1 nicht explizit dargestellt ist, so sind die Probenbearbeitungsvorrichtung 120 und die mikrofluidische Kartusche 110 fluidisch oder fluidisch und mechanisch miteinander koppelbar.

Die mikrofluidische Kartusche 110 ist ausgebildet, um die Probe vorzubereiten und der Probenbearbeitungsvorrichtung 120 die Zielmoleküle der Probe bzw. ein Resultat der Probenvorbereitung, beispielsweise aus Zellen extrahierte und aufgereinigte DNA, und Stoffe für das Analysieren der Probe zuzuführen. Die mikrofluidische Kartusche 110 weist einen Abschnitt zum Einbringen der Probe in das mikrofluidische System 100, vorgelagerte Reagenzien und eine Aktuierungseinheit auf, um der Amplifikationskammer und der Nachweiskammer mit den Kavitäten die Zielmoleküle der Probe und Stoffe für das Analysieren der Probe zuzuführen.

Die Probenbearbeitungsvorrichtung 120 ist aus einem Polymermaterial ausgeformt. Auch wenn es in Fig. 1 nicht erkennbar ist, weist die Probenbearbeitungsvorrichtung 120 eine Amplifikationskammer für Amplifikationsreaktionen an Zielmolekülen der Probe und eine fluidisch mit der Amplifikationskammer verbundene Nachweiskammer mit einer Mehrzahl von Kavitäten zum Ausführen von parallelen Nachweisreaktionen an den amplifizierten Zielmolekülen auf. Dabei sind die Nachweiskammer der Probenbearbeitungsvorrichtung 120 und die Kavitäten der Nachweiskammer für eine Verteilung der Zielmoleküle der Probe auf die Kavitäten ausgeformt.

Fig. 2 zeigt eine schematische Darstellung einer Probenbearbeitungsvorrichtung 120 gemäß einem Ausführungsbeispiel der vorliegenden Erfindung. Bei der Probenbearbeitungsvorrichtung 120 handelt es sich um die Probenbearbeitungsvorrichtung aus Fig. 1. Somit ist die in Fig. 2 dargestellte Probenbearbeitungsvorrichtung 120 als Probenbearbeitungsvorrichtung des mikrofluidischen Systems aus Fig. 1 anwendbar. In Fig. 2 sind von der Probenbearbeitungsvorrichtung 120 eine Zufuhröffnung 202, ein Einlassventil 204, eine Amplifikationskammer 210, ein Verbindungsventil 222, ein erstes Ausgangsventil 224, eine erste Ausgangsöffnung 226, eine Spülmedienzufuhröffnung 232, ein Spülmedieneinlassventil 234, eine Speicherkammer 240, ein weiteres Verbindungsventil 252, ein zweites Ausgangsventil 254, eine zweite Ausgangsöffnung 256, eine Nachweiskammer 260, ein Array bzw. eine Mehrzahl von Kavitäten 265, ein Auslassventil 272 und eine Abfuhröffnung 274 gezeigt. Bei der Probenbearbeitungsvorrichtung 120 handelt es sich um einen Chip, insbesondere mit mehreren Schichten, aus einem Polymermaterial.

Die Probenbearbeitungsvorrichtung 120 weist als Kammern die Amplifikationskammer 210, die Speicherkammer 240 und die Nachweiskammer 260 auf. Die Amplifikationskammer 210 ist für Amplifikationsreaktionen an Zielmolekülen der Probe vorgesehen. Die Speicherkammer 240 ist zum Speichern eines Spülmediums für die Nachweiskammer 260 ausgebildet. Die Nachweiskammer 260 weist die Mehrzahl von Kavitäten 265 zum Ausführen von Nachweisreaktionen an den amplifizierten Zielmolekülen auf. Die Amplifikationskammer 210 ist fluidisch mit der Nachweiskammer 260 verbunden. Auch die Speicherkammer 240 ist fluidisch mit der Nachweiskammer 260 verbunden.

Die Amplifikationskammer 210 ist über einen das Einlassventil 204 aufweisenden, ersten Fluidkanal mit der Zufuhröffnung 202 verbunden. Ferner ist die Amplifikationskammer 210 über einen das Verbindungsventil 222 aufweisenden, zweiten Fluidkanal mit der Nachweiskammer 260 verbunden. Auch ist die Amplifikationskammer 210 über einen das erste Ausgangsventil 224 aufweisenden, dritten Fluidkanal mit der ersten Ausgangsöffnung 226 verbunden. Der dritte Fluidkanal zweigt zwischen der Amplifikationskammer 210 und dem Verbindungsventil 222 von dem zweiten Fluidkanal ab. Die erste Ausgangsöffnung 226 und das erste Ausgangsventil 224 sind ausgebildet, um für die Amplifikationskammer 210 ein Entlüften, Befüllen und zusätzlich oder alternativ Ablassen von Resten und/oder Überschüssen zu ermöglichen.

Die Speicherkammer 240 ist über einen das Spülmedieneinlassventil 234 aufweisenden, vierten Fluidkanal mit der Spülmedienzufuhröffnung 232 verbunden. Ferner ist die Speicherkammer 240 über einen das weitere Verbindungsventil 252 aufweisenden, fünften Fluidkanal mit der Nachweiskammer verbunden. Auch ist die Speicherkammer 240 über einen das zweite Ausgangsventil 254 aufweisenden, sechsten Fluidkanal mit der zweiten Ausgangsöffnung 256 verbunden. Der sechste Fluidkanal zweigt zwischen der Speicherkammer 240 und dem weiteren Verbindungsventil 252 von dem fünften Fluidkanal ab. Die zweite Ausgangsöffnung 256 und das zweite Ausgangsventil 254 sind ausgebildet, um für die Speicherkammer 240 ein Entlüften, Befüllen und zusätzlich oder alternativ Ablassen von Resten und/oder Überschüssen zu ermöglichen.

Die Nachweiskammer 260 ist über einen das Auslassventil 272 aufweisenden, siebten Fluidkanal mit der Abfuhröffnung 274 verbunden. Die Mehrzahl von Kavitäten 265 sind in der Nachweiskammer 260 angeordnet. Insbesondere sind die Mehrzahl von Kavitäten 265 in einem Array angeordnet. Dabei sind die Kavitäten 265 der Nachweiskammer 260 für eine Aufnahme von weniger als zwei Zielmolekülen der Probe pro Kavität 265 ausgeformt. Die Kavitäten 265 weisen hierbei Abmessungen sowie eine Form auf, die geeignet sind, um die Aufnahme zumindest eines Moleküls pro Kavität 265 zu ermöglichen. Somit weist eine Kavität 265 Dimensionen auf, die größer als Dimensionen eines Moleküls sind.

Gemäß einem Ausführungsbeispiel sind die Kavitäten 265 ausgebildet, um ein kapillares Befüllen derselben mit der Probe zu bewirken. Dabei kann optional eine Oberfläche innerhalb der Kavitäten 265 hydrophil sein und kann eine Oberfläche der Nachweiskammer 260 außerhalb der Kavitäten 265 hydrophob sein. Zusätzlich oder alternativ sind gemäß einem Ausführungsbeispiel die Kavitäten 265 spitzwinklig zulaufend ausgeformt. Insbesondere weisen die Kavitäten 265 dabei einen Öffnungswinkel zwischen 5 Grad und 90 Grad auf.

Anders ausgedrückt weist die Probenbearbeitungsvorrichtung 120 bzw. der mikrofluidische Chip die Amplifikationskammer 210 für die Durchführung einer Amplifikationsreaktion, die Speicherkammer 240 für die Vorlagerung von beispielsweise Öl und die Nachweiskammer 260 auf, die viele Vertiefungen bzw. Kavitäten 265 für parallele Nachweisreaktionen enthält. Eine Reaktionsmischung in der Amplifikationskammer 210 ist so beschaffen, dass beispielsweise alle zu untersuchenden DNA-Motive amplifiziert werden. In jeder einzelnen Kavität 265 sind beispielsweise eine bis vier Nachweisreaktionen durchführbar und damit ausgewählte DNA-Motive nachweisbar.

Beispielhafte Abmessungen der Probenbearbeitungsvorrichtung 120 umfassen eine Dicke des Chips bzw. der Probenbearbeitungsvorrichtung 120 von 0,5 bis 5 Millimeter, einen Kanaldurchmesser der Fluidkanäle von 10 Mikrometer bis 3 Millimeter, ein Volumen der Kammern 210, 240 und 260 von 1 Kubikmillimeter bis 1000 Kubikmillimeter, ein Volumen der Kavitäten 265 von 1 Femtoliter bis 1 Mikroliter und einen Winkel an den Kavitäten 265 von 5 Grad bis 90 Grad.

In Betrieb wird eine zu untersuchende Zielmoleküllösung beispielsweise zusammen mit einem Amplifikationshilfsstoff bzw. Amplifikationssystem über die Zufuhröffnung 202 in die Amplifikationskammer 210 eingebracht. Ein Öl als Spülmedium wird über die Spülmedienzufuhröffnung 232 in die Speicherkammer 240 eingebracht. Während dieser beiden Vorgänge sind das erste Ausgangsventil 224 und das zweite Ausgangsventil 254 geöffnet, wobei das Verbindungsventil 222 und das weitere Verbindungsventil 252 geschlossen sind. Überschüssige Luft oder Flüssigkeit kann über die erste Ausgangsöffnung 226 und die zweite Ausgangsöffnung 256 entweichen. Nach dem Befüllen werden das erste Ausgangsventil 224 und das zweite Ausgangsventil 254 geschlossen und Reaktionsbedingungen für eine Amplifikationsreaktion in der Amplifikationskammer 210 geschaffen.

Dann werden durch Öffnen des Verbindungsventils 222 die amplifizierten Zielmoleküle Probe bzw. das Reaktionsprodukt oder Amplifikat von der Amplifikationskammer 210 auf die in der Nachweiskammer 260 angeordneten befindlichen Kavitäten 265 unter kapillarer Befüllung der Kavitäten 265 verteilt. Durch Öffnen des weiteren Verbindungsventils 252 wird das in der Speicherkammer 240 angeordnete Spülmedium bzw. Öl in die Nachweiskammer 260 eingeleitet. Dadurch wird eine über den Kavitäten 265 angeordnete bzw. verbliebene Flüssigkeit verdrängt und werden die Kavitäten 265 mit dem Öl verschlossen bzw. überdeckt. Überschüssiges Volumen an Zielmoleküllösung und/oder Spülmedium kann über das geöffnete Auslassventil 272 und die Abfuhröffnung 274 entweichen.

Dann werden das Verbindungsventil 222, das weitere Verbindungsventil 252 und das Auslassventil 272, welche die Nachweiskammer 260 flankieren, vor dem Start der Nachweisreaktion verschlossen. In den Kavitäten 265 werden parallele Nachweisreaktionen durchgeführt. Der Reaktionsverlauf ist in Echtzeit verfolgbar, wobei quantitative Informationen über eine initiale Menge der jeweiligen DNA-Motive verfügbar sind. Zusätzlich oder alternativ ist am Ende der Reaktion eine Messung durchführbar, wobei eine ja/nein-Aussage möglich ist.

Ein beispielhafter Prozessablauf mit Parametern für Vorbereitung und Analyse der Probe ist im Folgenden dargelegt. Ein Parameterraum für eine Amplifikationsreaktion, bei der es sich um eine Polymerase-Kettenreaktion (PCR, Polymerase Chain Reaction) handelt, umfasst eine Anzahl an Thermozyklen von 1 bis 20, Temperaturzyklen von 93°C bis 96°C für eine Denaturierung, von 72°C für eine Verlängerung, und von 54°C bis 65°C für eine Primeranbindung. Es ist auch möglich, den Schritt auf der Verlängerungstemperatur wegzulassen, wobei eine zweischrittige PCR ausgeführt wird. Ein Parameterraum für eine Nachweisreaktion, bei der es sich um eine PCR handelt, umfasst eine Anzahl an Thermozyklen von 20 bis 60, Temperaturzyklen von 93°C bis 96°C für eine Denaturierung, von 72°C für eine Verlängerung und von 54°C bis 65°C für eine Primeranbindung. Es ist auch möglich, den Schritt auf der Verlängerungstemperatur wegzulassen, wobei eine zweischrittige PCR ausgeführt wird. Eine exemplarische Anzahl an PCR-Reaktionen beträgt bei der ersten PCR, d. h. bei der Amplifikation, 1 bis 20 Reaktionen und bei der zweiten PCR, d. h. beim Nachweis, 1 bis 50 Reaktionen.

Somit lässt sich unter Bezugnahme auf die Figuren 1 und 2 ein Ausführungsbeispiel eines mikrofluidischen Systems 100 bestehend aus Kanälen, Ventilen und Kammern in bestimmter Anordnung und Ausformung beschreiben. Zunächst wird die zu untersuchende Probenlösung, die aufgereinigte Zielmoleküle aufweist, in die Amplifikationskammer 210 eingebracht und mittels einer Amplifikationsreaktion voramplifiziert. Bei dieser Reaktion können zwischen 5 und 100 verschiedene Abschnitte einer Nukleinsäure vervielfältigt werden. Dieses Voramplifikat wird in die Nachweiskammer 260 eingeleitet, in der eine Mehrzahl von spitzwinklig zulaufenden Kavitäten 265 angeordnet sind. In jeder Kavität 265 sind zwischen 1 und 4 Primer- und Sondenpärchen vorgelagert. Aufgrund der Form der Kavitäten 265 befüllen sich diese kapillar mit dem Voramplifikat und die vorgelagerten Primer und Sonden rehydrieren. Durch Einleiten von Öl in die Nachweiskammer 260 werden die einzelnen Kavitäten 265 verschlossen. Dies verdrängt überschüssiges Voramplifikat und verschließt die mit dem Voramplifikat befüllten Kavitäten 265. Nun können verschiedene DNA-Motive in Einzelreaktionen parallel oder hochparallel nachgewiesen werden. Aufgrund der Abmessungen der Kavitäten 265 kann eine digitale Nachweisreaktion, wie beispielsweise eine PCR, durchgeführt werden. Dabei sind die Primer und Sonden für ein genetisches Motiv nicht nur in einer Kavität 265, sondern jeweils in x₁, x₂, ... xₙ Kavitäten 265 angeordnet, wobei n << y gilt und y eine Gesamtanzahl an Kavitäten 265 repräsentiert.

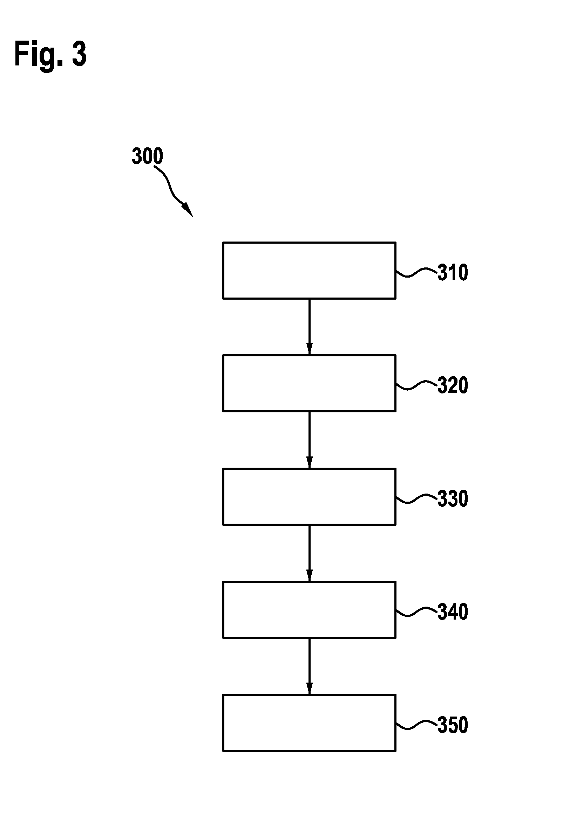

Fig. 3 zeigt ein Ablaufdiagramm eines Verfahrens 300 zum Analysieren einer Probe biologischen Materials gemäß einem Ausführungsbeispiel der vorliegenden Erfindung. Das Verfahren 300 ist in Verbindung mit einem mikrofluidischen System wie dem mikrofluidischen System aus Fig. 1 bzw. in Verbindung mit einer Probenbearbeitungsvorrichtung wie der Probenbearbeitungsvorrichtung aus Fig. 2 vorteilhaft ausführbar.

Das Verfahren 300 weist einen Schritt 310 des Bereitstellens einer Probenbearbeitungsvorrichtung aus einem Polymermaterial auf. Hierbei weist die Probenbearbeitungsvorrichtung eine Amplifikationskammer und eine fluidisch mit der Amplifikationskammer verbundene Nachweiskammer mit einer Mehrzahl von Kavitäten auf. Dabei sind die Nachweiskammer und die Kavitäten für eine Verteilung der Zielmoleküle der Probe auf die Kavitäten ausgeformt.

Auch weist das Verfahren 300 einen Schritt 320 des Zuführens von Zielmolekülen der Probe in die Amplifikationskammer auf. Zudem weist das Verfahren 300 einen Schritt 330 des Durchführens von Amplifikationsreaktionen an den in die Amplifikationskammer zugeführten Zielmolekülen auf. Ferner weist das Verfahren 300 einen Schritt 340 des Überführens der amplifizierten Zielmoleküle von der Amplifikationskammer in die Nachweiskammer auf. Dabei werden die Zielmoleküle der Probe auf die Kavitäten verteilt. Schließlich weist das Verfahren 300 einen Schritt 350 des Ausführens von parallelen Nachweisreaktionen an den amplifizierten, in die Kavitäten der Nachweiskammer überführten Zielmolekülen auf.

Gemäß einem Ausführungsbeispiel werden im Schritt 350 des Ausführens eine Mehrzahl von Nachweisreaktionen unter Verwendung von Wärme ausgeführt. Gemäß einem Ausführungsbeispiel wird im Schritt 320 des Zuführens zumindest ein Amplifikationshilfsstoff mit den Zielmolekülen der Probe in die Amplifikationskammer zugeführt. Gemäß einem Ausführungsbeispiel weist das Verfahren 300 zwischen dem Schritt 340 des Überführens und dem Schritt 350 des Ausführens von Nachweisreaktionen einen Schritt des Einleitens eines Spülmediums in die Nachweiskammer auf, um die mit den Zielmolekülen der Probe befüllten Kavitäten für die Nachweisreaktionen abzudecken. Gemäß einem Ausführungsbeispiel weist das Verfahren 300 vor dem Schritt 340 des Überführens einen Schritt des Anordnens zumindest eines Nachweishilfsstoffs in den Kavitäten auf. Dabei wird gemäß einem Ausführungsbeispiel der Schritt des Anordnens nach dem Schritt 310 des Bereitstellens durchgeführt oder ist der Schritt des Anordnens ein Teilschritt des Schrittes310 des Bereitstellens.

Die beschriebenen und in den Figuren gezeigten Ausführungsbeispiele sind nur beispielhaft gewählt. Unterschiedliche Ausführungsbeispiele können vollständig oder in Bezug auf einzelne Merkmale miteinander kombiniert werden. Auch kann ein Ausführungsbeispiel durch Merkmale eines weiteren Ausführungsbeispiels ergänzt werden.

Ferner können die hier vorgestellten Verfahrensschritte wiederholt sowie in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Mikrofluidisches System (100) zum Analysieren einer Probe biologischen Materials, wobei das mikrofluidische System (100) folgende Merkmale aufweist:
eine Probenbearbeitungsvorrichtung (120) aus einem Polymermaterial, wobei die Probenbearbeitungsvorrichtung (120) eine Amplifikationskammer (210) für Amplifikationsreaktionen an Zielmolekülen der Probe und eine fluidisch mit der Amplifikationskammer (210) verbundene Nachweiskammer (260) mit einer Mehrzahl von Kavitäten (265) zum Ausführen von Nachweisreaktionen an den amplifizierten Zielmolekülen aufweist, wobei die Nachweiskammer (260) und die Kavitäten (265) für eine Verteilung der Zielmoleküle der Probe auf die Kavitäten (265) ausgeformt sind; und
eine mit der Probenbearbeitungsvorrichtung (120) koppelbare mikrofluidische Kartusche (110) zum Vorbereiten der Probe und Zuführen der Zielmoleküle der Probe und von Stoffen für das Analysieren der Probe zu der Probenbearbeitungsvorrichtung (120).

2. Mikrofluidisches System (100) gemäß Anspruch 1, **dadurch gekennzeichnet, dass** die Kavitäten (265) ausgebildet sind, um ein kapillares Befüllen derselben mit den Zielmolekülen der Probe zu bewirken.

3. Mikrofluidisches System (100) gemäß einem der vorangegangenen Ansprüche, **dadurch gekennzeichnet, dass** die Kavitäten (265) spitzwinklig zulaufend ausgeformt sind.

4. Mikrofluidisches System (100) gemäß einem der vorangegangenen Ansprüche, **dadurch gekennzeichnet, dass** die Probenbearbeitungsvorrichtung (120) eine Zufuhröffnung (202), ein Einlassventil (204), ein Verbindungsventil (222), ein Auslassventil (272) und eine Abfuhröffnung (274) aufweist, wobei die Amplifikationskammer (210) über das Einlassventil (204) mit der Zufuhröffnung (202) verbunden ist, wobei die Amplifikationskammer (210) über das Verbindungsventil (222) mit der Nachweiskammer (260) verbunden ist, wobei die Nachweiskammer (260) über das Auslassventil (272) mit der Abfuhröffnung (274) verbunden ist.

5. Mikrofluidisches System (100) gemäß einem der vorangegangenen Ansprüche, **dadurch gekennzeichnet, dass** die Probenbearbeitungsvorrichtung (120) eine fluidisch mit der Amplifikationskammer (210) verbundene Speicherkammer (240) zum Speichern eines Spülmediums für die Nachweiskammer (260) aufweist.

6. Verfahren (300) zum Analysieren einer Probe biologischen Materials, wobei das Verfahren (300) folgende Schritte aufweist:
Bereitstellen (310) einer Probenbearbeitungsvorrichtung (120) aus einem Polymermaterial, wobei die Probenbearbeitungsvorrichtung (120) eine Amplifikationskammer (210) und eine fluidisch mit der Amplifikationskammer (210) verbundene Nachweiskammer (260) mit einer Mehrzahl von Kavitäten (265) aufweist, wobei die Nachweiskammer (260) und die Kavitäten (265) für eine Verteilung der Zielmoleküle der Probe auf die Kavitäten (265) ausgeformt sind;
Zuführen (320) der Zielmoleküle in die Amplifikationskammer (210);
Durchführen (330) von Amplifikationsreaktionen an den in die Amplifikationskammer (210) zugeführten Zielmolekülen;
Überführen (340) der amplifizierten Zielmoleküle von der Amplifikationskammer (210) in die Nachweiskammer (260), wobei die Zielmoleküle der Probe auf die Kavitäten (265) verteilt werden; und
Ausführen (350) von parallelen Nachweisreaktionen an den amplifizierten, in die Kavitäten (265) der Nachweiskammer (260) überführten Zielmolekülen.

7. Verfahren (300) gemäß Anspruch 6, **dadurch gekennzeichnet, dass** im Schritt (330) des Durchführens eine Amplifikationsreaktion unter Verwendung von Wärme ausgeführt wird, wobei im Schritt (350) des Ausführens eine Mehrzahl von Nachweisreaktionen unter Verwendung von Wärme ausgeführt werden.

8. Verfahren (300) gemäß einem der Ansprüche 6 bis 7, **dadurch gekennzeichnet, dass** im Schritt (320) des Zuführens zumindest ein Amplifikationshilfsstoff mit den Zielmolekülen in die Amplifikationskammer (210) zugeführt wird.

9. Verfahren (300) gemäß einem der Ansprüche 6 bis 8, **dadurch gekennzeichnet, dass** das Verfahren (300) zwischen dem Schritt (340) des Überführens und dem Schritt (350) des Ausführens von Nachweisreaktionen einen Schritt des Einleitens eines Spülmediums in die Nachweiskammer (260) aufweist, um die mit den Zielmolekülen befüllten Kavitäten (265) für die Nachweisreaktionen abzudecken.

10. Verfahren (300) gemäß einem der Ansprüche 6 bis 9, **dadurch gekennzeichnet, dass** das Verfahren (300) vor dem Schritt (340) des Überführens einen Schritt des Anordnens zumindest eines Nachweishilfsstoffs in den Kavitäten (265) aufweist.

11. Vorrichtung, die ausgebildet ist, um alle Schritte eines Verfahrens gemäß einem der Ansprüche 6 bis 10 durchzuführen und/oder anzusteuern.

12. Computerprogramm, das dazu eingerichtet ist, alle Schritte eines Verfahrens gemäß einem der Ansprüche 6 bis 10 durchzuführen und/oder anzusteuern.

13. Maschinenlesbares Speichermedium mit einem darauf gespeicherten Computerprogramm nach Anspruch 12.
